# EUROPEAN PATENT APPLICATION

(11) **EP 2 050 470 A1**
(43) Date of publication of application: **22.04.2009**
(21) Application number: 06741951.5
(22) Date of filing: 22.05.2006
(51) Int. Cl.: A61K 49/04

(54) **CT NEGATIVE CONTRAST MEDIUM OF AQUEOUS MATRIX FOR DIGESTIVE TRACT AND THE PREPARATION METHOD THEREOF**

(30) Priority: 11.05.2006 CN 200610026434
(71) Applicant: Shanghai Jiao Tong University, Minhang District Shanghai 200240 (CN)
(72) Inventor: WEI, Xiaohui, Shanghai 200240 (CN); XU, Jianrong, Shanghai 200001 (CN); XU, Yuhong, Shanghai 200240 (CN)
(74) Representative: Cabinet Plasseraud
(86) International application number: PCT/CN2006/001063
(87) International publication number: WO 2007/131390

(57) **Abstract**

An aqueous negative contrast agent for CT imaging of the gastrointestinal tract and the preparation method thereof. It comprises (weight percent): hydrogel matrix 0.01-1%, materials with low densities 5-50%, stabilizing agents 0.1-5%, and the rest is deionized water, wherein hydrogel is formed by cellulose and the derivatives thereof, agar, gelatin, acacia, tragacanth, chitosan and sodium polyacrylate. The low density materials comprise air, carbon dioxide, nitrogen, oxygen, fluoroalkanes, chlorofluoroalkanes, thiofluoroalkanes, inert gases and their combinations, or polymer particles of polyethylene and polypropylene. The stabilizing agent comprises proteins, caprolactone gluconate, ionic and nonionic surfactants, lipids, amphiphilic polymers and their combinations. The said contrast agent can help clearly present the intestine wall and the intestine lumen, can enhance the sensitivity and specificity of CT diagnosis for the diseases on the intestinal wall and intestinal lumen.

## Description

### Field of Invention

The invention relates to a contrast agent and the preparation method thereof. Particularly, the invention relates to an aqueous negative contrast agent for CT imaging of the gastrointestinal tract and the preparation method thereof. The invention is in the biomedical field.

### Background of the Invention

Diseases originated in the gastrointestinal tract (GI tract), especially malignant tumors such as the colon cancers pose daunting threats to human health. Computed Tomography (CT) is widely used for the medical imaging diagnosis of GI tract diseases. In recent years, with the application of the multi-planar spinal CT instrument with scanning speed at sub-second range, the GI imaging qualities have been greatly improved with much fewer artifacts resulted from breath and intestine peristalsis. Therefore CT plays a more and more important role in the diagnosis of GI tract diseases, especially in the diagnosis of colon diseases. In current clinical practice, air and water are usually used for the filling of the intestine to fully display the anatomic structures, in order to improve the detection accuracy and to reduce false positives. In addition, CT virtual endoscopy analysis based on three dimension (3D) image reconstruction was developed to further improve the diagnostic specificity and sensitivity for GI tract diseases. But the early and accurate detection of colon tumors that originated at the colon wall cannot be achieved by CT imaging. The most important reason for such a limitation is that imaging contrast agents currently available are either positive contrast agents (with high CT densities) or neutral contrast agents (with CT densities similar to that of water). The positive contrast agents tended to conceal the small changes on the intestinal wall, and the high CT density in the lumen may interfere with the colon wall images. The neutral contrast agents could not show clearly the anatomical structures of the intestinal wall, intestinal lumen and related tissues if without full filling of the colon, because the difference between their CT densities and that of the wall is not big enough. There is usually a high missing rate in diagnosis. Besides, the poor-contrast images cannot be used for virtual endoscopy reconstruction (VE and VR) and analysis. Therefore, it is important to develop a negative CT contrast agent (with low CT density) to efficiently improve intestinal wall visualization and 3D virtual endoscopy reconstruction.

So far, there is no such negative contrast agent available clinically that can satisfy all the three requirements in CT imaging for the GI tract: good filling of the intestine, optimal visualization of the intestinal wall and feasible 3D virtual endoscopy. There were reports proposing the use of plant cellulose, milk, fat emulsion, paraffin and air as the negative CT contrast agents. The CT densities of the plant cellulose, milk and fat emulsion are in the range of 10 to -80 Hounsfield Unit (HU), which is not low enough for high resolving power and the 3D virtual endoscopy reconstruction. The CT density of paraffin is around -100HU. It is useful for better depiction of the intestinal wall and 3D virtual endoscopy reconstruction. But severe diarrhea resulted from the administration of paraffin restricts its clinical application. Air filling has the CT density of -1000HU. The 3D virtual endoscopy reconstruction with air filling has higher sensitivity and specificity in detecting tumefied features in the lumen such as polyps or tumors. However, because of the air/water interface distortion, the depiction of the intestinal wall in the transverse images is poor and the thickness of the intestinal walls looks much thinner than normal, so it cannot accurately present the pathological changes on the intestinal wall.

Technological literature search shows that the only contrast agent that used in the clinic and actually improved the CT visualization of the intestinal wall was the Mucofalk suspension made by swelling plantain seeds. (Helical CT of the small bowel with an alternative oral contrast material in patients with Crohn disease, Doerfler OC, Ruppert-Kohlmayr AJ, Reittner P, et al, ABDOMINAL IMAGING, 2003, 28(3): 313), but the CT density difference between the intestinal wall and the lumen is still not large enough for 3D virtual endoscopy.

### Content of the Invention

The present invention addresses the problems and limitations of the contrast agents currently available for CT imaging of the intestinal tract and provides an aqueous negative contrast agent for CT imaging of the gastrointestinal tract and the preparation method thereof. The suspension-type negative contrast agent is safe, nontoxic and stable. It does not contain grease such as paraffine or vegetable oil, or other ingredients that may lead to diarrhea, such as mannitol. The intestine can be well filled with the negative contrast agent, the intestinal wall can be clearly visualized and the 3D virtual endoscopy can be carried out. Therefore, the sensitivity and the specificity of CT diagnosis of pathological changes on the intestinal wall and in the intestinal lumen are greatly improved. It provides a reliable radiographic basis for clinical diagnosis and treatment. Meanwhile, the aqueous negative contrast agent is convenient for administration. A complete series of radiographic checks can be carried out with a single preparation and contrast agent administration, so as to reduce the cost and the inconvenience for patients.

### The present invention is realized through the following technical solutions:

The aqueous negative contrast agent for CT imaging of the gastrointestinal tract of the present invention is a suspension composed of micro-nano-particles of low density materials suspended in hydrogel matrix. The components of the negative contrast agent and their weight concentrations are: hydrogel matrix 0.01-1%, micro-nano-particles of low density materials 5-50%, stabilizing agents 0.1-5%, and the rest is deionized water. The said micro-nano-particles of low density materials are gas micro-bubbles and/or solid particles with low densities.

The contrast agent of the present invention has the CT density in the range of - 30HU to -500HU. The contrast agent can substantially decrease the CT image density inside the intestine lumen, making it significantly lower than that of the intestinal wall after the full filling of the intestine, with uniform density distribution. The matrix of the contrast agent of the present invention is formulated into viscous solutions or semisolids by dispersing or swelling natural or synthetic hydrophilic polymers in water. The said negative contrast agent can fully fill the intestine while maintains good fluidity.

The present invention also provides the preparation method of the aqueous negative contrast agent for CT imaging of the gastrointestinal tract. Particularly, the method comprises: first of all, disperse or swell the natural or synthetic hydrophilic polymers in water to obtain the hydrogel matrix; add the stabilizing agents, add or prepare the low density micro-nano-particles to disperse them and to obtain the suspension in the hydrogel matrix. By adjusting the stabilizing agent and the viscosity of the hydrogels, the stability and the fluidity of the suspension is adjusted, and a negative contrast agent with good uniformity, stability and fluidity is achieved, which meets the requirements of the research of CT imaging of intestinal tract.

The low density materials used in this invention refer to materials that have densities lower than that of water, with CT densities in the range of -50HU to - 1000HU. They are easy to be dispersed and not soluble in water. These materials include: various gas microbubbles, polyethylene (PE) or polypropylene (PP) particles qualified for medical use, etc, or mixtures of the above with certain ratios. The concentration of the micro-nano-particles of the materials with low densities in the suspension can be adjusted according to application requirements. Generally, the concentration is 5-50%.

The gas microbubbles described in the present invention refer to gas microbubbles at 25°C under one atmospheric pressure. The gas can be: air, carbon dioxide, nitrogen, oxygen, fluoroalkanes, chlorofluoroalkanes, thiofluoroalkanes and inert gases including helium, neon, argon, krypton, xenon, and their combinations. The volume concentration of the gas microbubbles in the hydrogel matrix is 5-50% at 25°C under one atmospheric pressure.

The low CT density solid particles described in the present invention refer to polymer particles that are not soluble in water, with lower density than that of water and CT density in the range of -50HU to -1000HU at 25°Cunder one atmospheric pressure. The particles include polyolefin, polyethylene, polypropylene, their mixed polymers, and the combinations thereof. The diameters of the particles are in the range of 0.05microns to 1000microns. Its concentration in the hydrogel matrix is 5-50%.

The hydrogel matrix used in the present invention may be any type of the accepted natural and synthetic hydrophilic polymers in this technical field. It can be: cellulose and the derivatives thereof, chitosan and the derivatives thereof, agar, gelatin, acacia, tragacanth, sodium polyacrylate and their mixtures. The concentration of the hydrogel is 0.01-1%. Stabilizing agents should be further added into the suspension to increase the stability of the suspension, and to achieve a more uniform dispersion of the particles of the materials with low densities in the suspension.

The stabilizing agents used in the present invention are selected from proteins, caprolactone gluconate, ionic and nonionic surfactants, lipids, amphiphilic polymers and their combinations in different ratios. The examples are sodium dodecylsulphonate, sorbitol fatty acid ester, sorbitan fatty acid ester, copolymers of polyoxyethylene and polyoxypropylene (PEO-PPO), etc., and their combinations. The concentration of the stabilizing agents in the suspension is 0.1-5%.

There are two different methods for the preparation of the suspensions of gas microbubbles with low densities in the hydrogel matrix, depending on different preparations of the gas microbubbles. In one embodiment, gas, such as air, nitrogen and inert gases, is fed into the hydrogel matrix that contains stabilizing agents of certain concentrations, to directly generate gas microbubbles by high speed stirring. The gas microbubbles are further dispersed and stabilized with the aid of the stabilizing agents. In the other embodiment, liquids with low boiling points, such as fluoroalkane, chlorofluoroalkane and thiofluoroalkane and their mixtures, are emulsified into the hydrogel matrix at the temperatures below the phase-transition temperatures to form an oil-in-water emulsion. The gas microbubbles coated with the stabilizing agents are generated by heating the said oil-in-water emulsion.

The suspensions of the low CT density solid particles in the hydrogel matrix are prepared by any of the three following methods. In one embodiment, the pre-frozen particles of polyethylene or polypropylene for medical use are loaded into a jet pulverizer for micronization. The resulted fine particles with diameters of 0.05-1000 microns are further mixed with the stabilizing agents, and dispersed into the hydrogel matrix to obtain low density solid particle suspensions with different CT densities. In another embodiment, the particles of polyethylene or polypropylene for medical use are mixed with the stabilizing agents, pre-frozen, and loaded into a jet pulverizer for micronization. The resulted fine particles with diameters of 0.005-1000 microns and hydrophilic surfaces are then dispersed into hydrogel matrix to obtain suspensions of solid particles with low densities. In the third embodiment, the stabilizing agents are dissolved in organic solvents, and sprayed uniformly onto the surfaces of the particles of polyethylene or polypropylene for medical use. Solid granules are resulted by granulation and removal of the organic solvents. The low density solid suspensions with different CT densities are prepared by dispersing the solid granules into the hydrogel matrix prior to administration.

The aqueous negative contrast agent for CT imaging of the intestinal tract as prepared in the present invention is of good uniformity, stability and fluidity. It can effectively enhance the definition and contrast of the intestinal wall images. Low density gas microbubbles and/or low density solid particles are uniformly dispersed in the suspensions without separation. In addition, the said negative contrast agent has good fluidity, which meets the requirement for clinical enema administration. In CT imaging studies on ex vivo pig small intestines and in Beagle dogs gastrointestinal tract with water as control group, the aqueous negative contrast agent were shown to greatly decrease the CT density of the intestine lumen to as low as - 30HU to -200HU. Because the CT density outside intestines is around -100HU to - 150HU, the resolution, completeness and smoothness of the intestine walls shown in the CT images using the aqueous negative contrast agent are much better than those in the control group using water instead of the agent of the present invention. Besides, the signals of the intestine lumens are uniform. There is no signal difference resulted from the low density material particles aggregation or separation. These further enhance the reliability of the imaging diagnosis.

The aqueous negative contrast agent for CT imaging of the intestinal tract as prepared in the present invention can at the same time solve the problems on the 2D intestine wall depiction and 3D virtual endoscopy reconstruction. Thus, the sensitivity and specificity for imaging diagnosis of diseases originating from the intestine wall and lumen are greatly improved. This is especially significant for the early reliable detection of the malignant tumors such as colon cancer. Hydrogel is used as the matrix for the prepared negative contrast agent. It can fill the intestine well, and eliminate the severe diarrhea resulted from the administration of oils, fats, mannitol, etc. The gas microbubbles and/or solid particles with low densities are safe and non-irritant. They can effectively decrease the CT density of the lumen after enema administration into the intestine. The materials for the preparation of the aqueous negative contrast agent are commonly available. The preparation method is simple and easy to be carried out, suitable for mass production. According to different imaging requests, the CT density of the aqueous negative contrast agent can be easily adjusted by changing the added amount of the materials with low CT densities in the hydrogel matrix for clear 2D intestine wall depiction and good 3D images reconstruction.

The examples according to the present invention are as follows, which further illustrate the technical solution of the present invention.

### Example 1

In this example, the major components of the contrast agent are: polyethylene particles for medical use with mean diameter of 200 microns prepared by ultra-fine milling which accounts for 20%, the mixture of Pluronic F68 and sodium dodecylsulphonate as the stabilizing agent which accounts for 5%, sodium polyacrylate which accounts for 0.01%, the rest is deionized water. The preparation method is as follows: commercial available polyethylene particles for medical use and Pluronic F68 are mixed by the ratio of 100:1, then loaded to jet pulverizer for ultra-fine milling. Polyethylene particles with the mean diameter of 200 microns, and with Pluronic F68 absorbed on the surface are resulted. 20g resulted polyethylene particles and 5g stabilizing agent (the mixture of 1g SDS and 4g Pluronic F68) are mixed in a mortar. Then, 20ml pre-swelled sodium polyacrylate (with Mw higher than 300,000) hydrogel with the concentration of 0.05% is added into the mortar, and mixed well. Deionized water is added into the mortar until the total weight of the suspension reaches 100g. The suspension is transferred to a flat-bottom beaker, and further blended by magnetic stir at medium speed. Uniform suspension with polyethylene particles suspended in sodium polyacrylate hydrogel matrix is obtained.

The measurement of the CT density of the contrast agent: the prepared suspension is loaded into plastic tubes with caps, and CT scanning is performed for the measurement of its CT density. The measurement results indicate that: in the control group, the CT density of water is zero; the CT density of the prepared negative contrast agent suspension is -30HU. There is no significant difference in the CT densities among different scanning layers. The suspension keeps stable for 20min without separation.

The measurement of the intestine lumen CT densities in ex vivo pig intestines is as follows: One piece of the ex vivo pig intestine was filled with the prepared suspension. Another intestine was filled with water. The two pieces of the ex vivo pig intestines are immersed in paraffine or vegetable oil that simulate the fat environment outside intestine in vivo. This is also helpful to shield the interference from the gas interface. CT scanning is performed and the CT densities of the intestine lumens are measured. The results are: the CT density of the intestine lumen in the control group is around 0HU, and the intestine wall depiction is poor; the CT density of the intestine lumen in the study group which is filled with the negative contrast agent is around -30HU, and the intestine wall depiction is significantly better than the control group. The signals densities inside the lumen are uniform.

### Example 2

In this example, the major components of the contrast agent are: polyethylene particles for medical use with mean diameter of 10 microns prepared by co-milling which accounts for 50%, the mixture of Pluronic F68 and SDS as the stabilizing agent which accounts for 3.5%, sodium polyacrylate which accounts for 0.005% and methylcellulose which accounts for 0.03%. The rest is deionized water. The preparation method is as follows: the polypropylene particles for medical use with CT density of -200HU which is sieved through 40-mesh sifter are mixed with Pluronic F68 by the ratio of 100:1, and then frozen. The mixture is then loaded into and co-milled by an air muller to get polypropylene particles with the mean diameter of 10 microns and with hydrophilic surface. 50g resulted polypropylene particles and 3.5g stabilizing agent (comprising 0.5g SDS and 3g Pluronic F68) are mixed in a mortar. Then, 0.05% sodium polyacrylate solution and 0.3% methyl cellulose solution both in 10ml are added into the mortar, and mixed well. Deionized water is added into the mortar until the total weight of the suspension reaches 100g. The suspension is further blended by magnetic stir. The CT density of the resulted aqueous negative contrast agent with polypropylene particles suspended in is measured using the method as described in example 1, as well as the pig intestine lumen CT density after filling with the agent. The results are: the CT density of the prepared negative contrast agent is around -100HU; the CT density of the pig intestine lumen is decreased to as low as -100HU. The intestine wall is visualized clearly, completely and smoothly. The signals intensities inside the lumen are uniform without visible conglomeration in the CT images.

### Example 3

In this example, the major components of the contrast agent are: polypropylene particles for medical use with mean diameter of 1000 microns which accounts for 35%, the mixture of Pluronic F68 and sodium dodecylsulphonate as the stabilizing agent which accounts for 4.8%; agar which accounts for 0.005% and methylcellulose which accounts for 0.02%. The rest is deionized water. The preparation method is as follows: 100g ultra-fine milled PP particles for medical use with the mean diameter of 1000 microns and with CT density of -100HU are put into a coating pan. Pluronic F68 ethanol solution with concentration of 6% is loaded into the sprayer and is uniformly sprayed onto the surface of the PP particles. The polypropylene particles coated with Pluronic F68 are taken out, dried at 60°C and weighed. Make sure the Pluronic F68 coated on the polypropylene particles surface is 2%. 35g dried polypropylene particles and 4g stabilizing agent (comprising 0.5g sodium dodecylsulphonate and 3.5g Pluronic F68) are loaded and mixed well in a mortar. Then, 0.05% agar solution, and 0.2% methyl cellulose solution, both in 10ml, and deionized water of adequate amount are added into the mortar, and mixed well. Deionized water is added into the mortar until the total weight of the suspension reaches 100g. The suspension is further blended by magnetic stir at medium speed. The CT density of the resulted aqueous negative contrast agent with polypropylene particles suspended in is measured using the method as described in example 1, as well as the pig intestine lumen CT density after filled with the agent. The results are:
the CT density of the prepared negative contrast agent is around -85HU; the CT density of the pig intestine lumen is decreased to as low as -85HU. The intestine wall is visualized clearly, completely and smoothly. The signals intensities inside the lumen are uniform without visible conglomeration in the CT images.

### Example 4

In this example, the major components of the contrast agent are: fine powders of surface modified polyethylene and polypropylene mixture for medical use with mean diameter of 50 nanometer which accounts for 5%, the mixture of the block copolymer of PEO-PPO and egg lecithin as the stabilization agent account for 2%,tragacanth which accounts for 0.5%, and the rest is deionized water. The preparation method is as follows: particles of polyethylene and polypropylene mixture for medical use, lecithin and the PEO-PPO block copolymer and egg lecithin are mixed in the ratio of 50:1:1, and then loaded to jet pulverizer for ultra-fine milling. Fine powders of polyethylene and polypropylene mixture with the mean diameter of 50 nanometers and with the stabilizing agents adsorbed on the surface are resulted. The CT density of the resulted fine powders of polyethylene and polypropylene mixture is -1000HU. 5g resulted polyethylene and polypropylene mixture fine powders and 2g PEO-PPO block copolymer are added into 0.5% tragacanth solution to form polypropylene tragacanth gel suspension with the total weight of 100g. The prepared polyethylene-polypropylene mixture negative contrast agent is tested using the method to measure the CT density of negative contrast agent and the CT density in the filled intestine lumen as described in example 1. The results are: the CT density of the prepared negative contrast agent is around -200HU; the CT density of the pig intestine lumen is decreased to as low as -200HU. The intestine wall is visualized clearly, completely and smoothly. The signals intensities inside the lumen are uniform without visible conglomeration in the CT images.

### Example 5

In this example, the major components of the contrast agent are: fine powders of the surface modified ethylene-propylene random copolymer with mean diameter of 500 nanometer which accounts for 10%, the mixture of cremophor RH and Tween as the stabilizing agent which accounts for 3%, acacia which accounts for 0.2%, and the rest is deionized water. The preparation method is as follows: particles of ethylene-propylene random copolymer, cremophor RH and Tween 60 are mixed by the ratio of 100:1:1, and then loaded to jet pulverizer for ultra-fine milling. Fine powders of ethylene-propylene random copolymer with the mean diameter of 100 nanometers and with the stabilizing agents cremophor RH and Tween adsorbed on the surface in are resulted. The CT density of the resulted fine powders of ethylene-propylene random copolymer is -600HU. 10g resulted fine powders of ethylene-propylene random copolymer, 1g cremophor RH and 2g Tween are added into 0.2% acacia hydrogel to form ethylene-propylene random copolymer acacia suspension with the total weight of 100g. The prepared polypropylene-methylcellulose hydrogel negative contrast agent is tested using the method to measure the CT density of negative contrast agent and the CT density in the filled intestine lumen as described in example 1. The results are: the CT density of the prepared negative contrast agent is around -100HU; the CT density of the pig intestine lumen is decreased to as low as -100HU. The intestine wall is visualized clearly, completely and smoothly. The signals intensities inside the lumen are uniform without visible conglomeration in the CT images.

### Example 6

In this example, the major components of the contrast agent are: fine powders of the surface modified ethylene-propylene block copolymer with mean diameter of one micron which accounts for 40%, the mixture of cremophor RH, Tween and Span as the stabilizing agent which accounts for 5%, sodium carboxylmethyl cellulose (CMC-Na) which accounts for 0.8%, and the rest is deionized water. The preparation method is as follows: particles of ethylene-propylene block copolymer, cremophor RH, Tween and Span are mixed by the ratio of 100:1:2:1, and then loaded to jet pulverizer for ultra-fine milling. Fine powders of ethylene-propylene block copolymer with the mean diameter of one micron and with the stabilization agents adsorbed on the surface are resulted. The CT density of the resulted fine powders of ethylene-propylene block copolymer is -400HU. 40g resulted fine powders of ethylene-propylene block copolymer, 1g cremophor RH, 2g Tween and 2g Span are added into 0.8% CMC-Na hydrogel to form ethylene-propylene block copolymer suspension with the total weight of 100g. The prepared polypropylene-methylcellulose hydrogel negative contrast agent is tested using the method to measure the CT density of negative contrast agent and the CT density in the filled intestine lumen as described in example 1. The results are: the CT density of the prepared negative contrast agent is around -80HU; the CT density of the pig intestine lumen is decreased to as low as -80HU. The intestine wall is visualized clearly, completely and smoothly. The signals intensities inside the lumen are uniform without visible conglomeration in the CT images.

### Example 7

In this example, the major components of the contrast agent are: air microbubbles generated via high speed stir which accounts for 20%(v/v), albumin and caprolactone gluconate as the stabilizing agent which accounts for 0.1%, methylcellulose which accounts for 0.4%, agar which accounts for 0.1 %, gelatin which accounts for 0.05%, and the rest is deionized water. The air microbubbles are stabilized by being linked with the stabilizing agent in the hydrogel matrix. The preparation method is as follows: 0.1g stabilizing agent (comprising 0.05g albumin and 0.05g caprolactone gluconate) is added into 100ml hydrogel composed of 0.4% methylcellulose, 0.1% agar and 0.05% gelatin, and mixed well. Milky air microbubble-hydrogel suspension is prepared by stir in a homogenizer at the speed of 10000rpm for 5min. The prepared air microbuble-hydrogel negative contrast agent is tested using the method to measure the CT density of negative contrast agent and the CT density in the filled intestine lumen as described in example 1. The results are: the CT density of the prepared negative contrast agent is around -200HU; the CT density of the pig intestine lumen is decreased to as low as -200HU. The signals intensities inside the lumen are uniform without visible conglomeration in the CT images.

### Example 8

In this example, the major components of the contrast agent are: nitrogen microbubbles which account for 50%(v/v), the mixture of albumin, cetyltrimethyl ammonium bromide (CTAB) and PEO-PPO block copolymers as the stabilizing agent which accounts for 1.55%, chitosan which accounts for 0.5%, gelatin which accounts for 0.5%, and the rest is deionized water. The nitrogen microbubbles generated via high speed stir under nitrogen stream feeding are stabilized with the stabilizing agent in the hydrogel matrix. The preparation method is as follows: 1.55g stabilizing agent (comprising 1g albumin, 0.05g CTAB and 0.5g Pluronic F68) is added into 100ml hydrogel composed of 0.5% chitosan and 0.5% gelatin, and mixed well. Milky nitrogen microbubble-hydrogel suspension is prepared by stir in a homogenizer at the speed of 10000rpm for 5min under continuous feeding of nitrogen. The prepared nitrogen microbubble-hydrogel negative contrast agent is tested using the method to measure the CT density of negative contrast agent and the CT density in the filled intestine lumen as described in example 1, as well as the pig intestine lumen CT density after filling with the agent. The results are: the CT density of the prepared negative contrast agent is around -500HU; the CT density of the pig intestine lumen is decreased to as low as -500HU. The signals intensities inside the lumen are uniform without visible conglomeration in the CT images.

### Example 9

In this example, the major components of the contrast agent are: helium microbubbles which account for 5%(v/v), the mixture of sodium dodecylsulphonate (SDS) and Pluronic F68 as the stabilizing agent which accounts for 1%, methylcellulose which accounts for 0.4%, agar which accounts for 0.3%, and the rest is deionized water. The helium microbubbles generated via high speed stir under helium stream feeding are stabilized with the stabilizing agent in the hydrogel matrix. The preparation method is as follows: 1g stabilizing agent (comprising 0.5g SDS and 0.5g Pluronic F68) is added into 100ml mixed hydrogel composed of 0.4% methylcellulose and 0.3% agar, and mixed well. Milky helium microbubble-hydrogel suspension is prepared by stir in a homogenizer at the speed of 10000rpm for 3min under continuous feeding of helium. The prepared helium microbubble-hydrogel negative contrast agent is tested using the method to measure the CT density of negative contrast agent and the CT density in the filled intestine lumen as described in example 1. The results are: the CT density of the prepared negative contrast agent is around -50HU; the CT density of the pig intestine lumen is decreased to as low as -50HU. The signals intensities inside the lumen are uniform without visible conglomeration in the CT images.

### Example 10

In this example, the major components of the contrast agent are: SF₆ microbubbles which account for 10%(v/v), the mixture of phospholipid, phosphatidic acid and Pluronic F68 as the stabilizing agent which accounts for 2%, methylcellulose which accounts for 0.3%, agar which accounts for 0.2%, and the rest is deionized water. The SF₆ microbubbles generated via heating gasification are stabilized with the stabilizing agent in the hydrogel matrix. The preparation method is as follows: 2g stabilizing agent (comprising 0.8g Pluronic F68, 1g phospholipid and 0.2g phosphatidic acid) is added into 100ml hydrogel composed of 0.3% methylcellulose and 0.2% agar, and mixed well. The solution is cooled down by ice cold bath, and SF₆ in liquid state is dropped in. Oil-in-water emulsion with SF₆ as the oil phase is prepared by stir in a homogenizer at the speed of 10000rpm for 1min. The emulsion is heated by a water bath at 25°C, and SF₆ is gasifies to form gas microbubbles. Milky SF₆ microbubble-hydrogel suspension is obtained. The prepared SF₆ microbubble-hydrogel negative contrast agent is tested using the method to measure the CT density of negative contrast agent and the CT density in the filled intestine lumen as described in example 1. The results are: the CT density of the prepared contrast agent is around -290HU; the CT density of the pig intestine lumen is decreased to as low as -290HU. The signals intensities inside the lumen are uniform without visible conglomeration in the CT images.

### Example 11

In this example, the major components of the contrast agent are: trichlorofluoromethane microbubbles which account for 30%(v/v), the mixture of phophatidylethanolamine, phosphatidic acid and Pluronic F68 as the stabilizing agent which accounts for 5%, sodium carboxylmethyl cellulose (CMC-Na) which accounts for 0.1%, agar which accounts for 0.1%, and the rest is deionized water. The trichlorofluoromethane microbubbles generated via heating gasification are stabilized with the stabilizing agent in the hydrogel matrix. The preparation method is as follows: 5g stabilizing agent (comprising 2.5g Pluronic F68, 2g phosphatidylethanolamine and 0.5g phosphatidic acid) is added into 100ml hydrogel composed of 0.1% CMC-Na and 0.1% agar, and mixed well. The solution is cooled down by ice cold bath, and trichlorofluoromethane in liquid state is dropped in. Oil-in-water emulsion with trichlorofluoromethane as the oil phase is prepared by stir in a homogenizer at the speed of 10000rpm for 3min. The emulsion is heated by a water bath at 30°C, and trichlorofluoromethane is gasifies to form gas microbubbles. Milky trichlorofluoromethane micububble-hydrogel suspension is obtained. The prepared trichlorofluoromethane microbubble-hydrogel negative contrast agent is tested using the method to measure the CT density of negative contrast agent and the CT density in the filled intestine lumen as described in example 1. The results are: the CT density of the prepared contrast agent is around -400HU; the CT density of the pig intestine lumen is decreased to as low as -400HU. The signals intensities inside the lumen are uniform without visible conglomeration in the CT images.

### Example 12

In this example, the major components of the contrast agent are: microbubbles of the mixture of dichlorofluoroethane and trichlorofluoromethane which account for 20%(v/v), the mixture of phospholipid, phophatidylethanolamine, and Pluronic F68 as the stabilizing agent which accounts for 3%, sodium polyacrylate which accounts for 0.05%, agar which accounts for 0.1%, and the rest is deionized water. The microbubbles of the dichlorofluoroethane and trichlorofluoromethane mixture generated via heating gasification are stabilized with the stabilizing agent in the hydrogel matrix. The preparation method is as follows: 3g stabilizing agent (comprising 1.5g Pluronic F68, 0.5g phosphatidylethanolamine and 1g phospholipid) is added into 100ml hydrogel composed of 0.05% sodium polyacrylate and 0.1% agar, and mixed well. The solution is cooled down by ice cold bath, and dichlorofluoroethane and trichlorofluoromethane mixture is added in. Oil-in-water emulsion with dichlorofluoroethane and trichlorofluoromethane mixture as the oil phase is prepared by stir in a homogenizer at the speed of 10000rpm for 3min. The emulsion is heated by a water bath at 40°C, and dichlorofluoroethane and trichlorofluoromethane mixture is gasified to form gas microbubbles. Milky micububbles of dichlorofluoroethane and trichlorofluoromethane mixture-hydrogel suspension is obtained. The prepared gas microbubble-hydrogel negative contrast agent is tested using the method to measure the CT density of negative contrast agent and the CT density in the filled intestine lumen as described in example 1. The results are: the CT density of the prepared negative contrast agent is around -250HU; the CT density of the pig intestine lumen is decreased to as low as -250HU. The signals intensities inside the lumen are uniform without visible conglomeration in the CT images.

## Claims

**1.** An aqueous negative contrast agent for CT imaging of the gastrointestinal tract, **characterized in that** the said contrast agent is a suspension of micro-nano-particles of low density materials suspended in hydrogel matrices, comprising (weight percentage): hydrogel matrix 0.01-1%, low density micro-nano-particles 5-50%, stabilizing agents 0.1-5%, and water, wherein the said micro-nano-particles of low density materials are low density gas microbubbles and/or low density solid particles.

**2.** The aqueous negative contrast agent for CT imaging of the gastrointestinal tract according to Claim 1, **characterized in that** the said suspension has the CT densities in the range of -30HU to -500HU.

**3.** The aqueous negative contrast agent for CT imaging of the gastrointestinal tract according to Claim 1, **characterized in that** the matrix of the said suspension is a viscous liquid or semisolid formed by natural or synthetic hydrophilic polymers which are dispersed or swelled in water.

**4.** The aqueous negative contrast agent for CT imaging of the gastrointestinal tract according to Claim 1, **characterized in that** the said hydrogel matrix comprises:
cellulose and the derivatives thereof, agar, gelatin, acacia, tragacanth, chitosan and
the derivatives thereof, sodium polyacrylate and their combinations of different ratios, wherein the concentration of the hydrogel is 0.01-1%.

**5.** The aqueous negative contrast agent for CT imaging of the gastrointestinal tract according to Claim 1, **characterized in that** the said low density gas microbubbles are microbubbles at 25°Cunder 1atm, comprising: air, carbon dioxide, nitrogen, oxygen, fluoroalkanes, chlorofluoroalkanes, thiofluoroalkanes, inert gases including helium, neon, argon, krypton, xenon, and their combinations, wherein the volume concentration of the gas microbubbles in the hydrogel matrix is 5-50% at 25°C under 1atm.

**6.** The negative contrast agent of aqueous matrix for CT imaging of the gastrointestinal tract according to Claim 1, **characterized in that** the said low density solid particles are water insoluble polymer particles with densities lower than that of water at 25 °C under 1atm which have CT values between -50HU to -1000HU, comprising polyolefin, polyethylene, polypropylene, their mixed polymers, and the combination thereof, wherein the diameters of the solid particles are in the range of 0.05microns to 1000microns, and their concentration in the hydrogel matrix is 5-50%.

**7.** The aqueous negative contrast agent for CT imaging of the gastrointestinal tract according to Claim 1, **characterized in that** the said stabilizing agent is selected from at least one of the group comprising proteins, caprolactone gluconate, ionic and nonionic surfactants, lipids, amphiphilic polymers and their combinations of different ratios.

**8.** The method for preparing the aqueous negative contrast agent for CT imaging of the gastrointestinal tract according to Claim 1, **characterized in that:** add stabilizing agent into the hydrogel matrix, add or prepare the gas microbubbles with low density and/or solid particles with low density, and uniformly disperse by external force to obtain the aqueous negative contrast agent for CT imaging of the gastrointestinal tract.

**9.** The method for preparing the aqueous negative contrast agent for CT imaging of the gastrointestinal tract according to Claim 8, **characterized in that:** the said gas microbubbles with low density are generated by mechanical forcing and being uniformly dispersed in the hydrogel under gas stream feeding.

**10.** The method for preparing the aqueous negative contrast agent for CT imaging of the gastrointestinal tract according to Claim 8, **characterized in that**: the said gas microbubbles with low density are prepared as follows: fluoroalkane, chlorofluoroalkanes, thiofluoroalkanes, or their combinations are dispersed into the hydrogel matrix at the temperatures below their phase-transition temperatures to form oil-in-water emulsions; gas microbubbles of fluoroalkane, chlorofluoroalkanes, thiofluoroalkanes, or their combinations are gasified by heating the oil-in-water emulsion.

**11.** The method for preparing the aqueous negative contrast agent for CT imaging of the gastrointestinal tract according to Claim 8, **characterized in that,** the said low density solid particles are prepared by milling or fine milling, with hydrophilic surface modification before or after milling or fine milling.
